# EUROPEAN PATENT APPLICATION

(11) **EP 3 590 519 A1**
(43) Date of publication of application: **08.01.2020**
(21) Application number: 18761539.8
(22) Date of filing: 26.02.2018
(51) Int. Cl.: A61K 35/20, A61K 38/08, A61K 38/10, A61P 1/14, A61P 3/04, A61P 3/10, A61P 43/00

(54) **GLP-1 SECRETAGOGUE AND COMPOSITION**

(30) Priority: 03.03.2017 JP 2017040867
(71) Applicant: Morinaga Milk Industry Co., Ltd., Minato-ku Tokyo 108-8384 (JP)
(72) Inventor: HIRA, Tohru, Sapporo-shi Hokkaido 060-0808 (JP); HARA, Hiroshi, Sapporo-shi Hokkaido 060-0808 (JP); KOMATSU, Yosuke, Zama-shi Kanagawa 252-8583 (JP); IZUMI, Hirohisa, Zama-shi Kanagawa 252-8583 (JP)
(74) Representative: Vossius & Partner Patentanwälte Rechtsanwälte mbB
(86) International application number: PCT/JP2018/007005
(87) International publication number: WO 2018/159546

(57) **Abstract**

The present invention addresses the problem of providing an exceptional GLP-1 secretagogue that is a component derived from milk, a composition for promoting GLP-1 secretion, and a food/beverage or drug containing the same. Any selected from the group consisting of micellar casein, a hydrolysate thereof, a fraction thereof, a purified product thereof, and peptides including an amino acid sequence indicated by any of SEQ ID NOS.: 1-7 is configured as an active ingredient of a GLP-1 secretagogue and a composition for promoting GLP-1 secretion.

## Description

### Technical Field

The present invention relates to a gastrointestinal hormone glucagon-like peptide-1 (GLP-1) secretagogue and a composition for promoting GLP-1 secretion. The composition for promoting GLP-1 secretion can be used as a drug, a food or drink, or a feed.

### Background Art

GLP-1 is one of glucagon-related gastrointestinal hormones and has an incretin effect which is important for glucose metabolism.

GLP-1 is released into the blood on taking meals from L-cells, which are gastrointestinal endocrine cells existing mainly in the lower small intestine, and influences pancreatic beta cells in a blood-glucose-concentration-dependent manner to promote secretion of insulin, thus reducing the postprandial blood glucose level (NPL 1). Furthermore, GLP-1 suppresses glucagon secretion, thereby decreasing release of glucose from the liver to reduce the blood glucose level. Besides, GLP-1 is reported to act on the central nervous system, thereby suppressing appetite (NPL 2).

Since such actions of GLP-1 are useful for improving diabetes and other lifestyle-related diseases, a GLP-1 analogue (GLP-1 receptor agonist) or an inhibitor of dipeptidyl peptidase IV (DPP-IV) which is a blood GLP-1-degrading enzyme is used in treatment of diabetes or the like.

A GLP-1-releasing activity is also being studied and there are many reports about GLP-1-releasing activity of proteins in addition to sugars or lipids (NPLs 3 to 8). The following mechanism is suggested: ingested proteins are degraded by digestive enzymes into peptides or amino acids, and then act on the peptide transporter 1 (PEPT 1) of a proton co-transport type or a calcium-sensing receptor (CaSR) on GLP-1-producing cells which are mainly located in the lower gastrointestinal tract, or activate intracellular signaling of intracellular calcium signals, mitogen-activated protein kinase (MAPK), or the like, thus promoting the GLP-1 secretion. (NPLs 9 to 11).

The GLP-1-releasing activity of milk or milk-derived proteins is also studied. For example, it is reported that in a human clinical test, the GLP-1 concentration in the blood plasma is increased on ingested cow's milk (NPL 12). It is also reported that casein glycomacropeptide (CGMP) obtained from hydrolyzed milk by rennet has a GLP-1-releasing activity in the small intestinal cell line (PTL 1). In addition, acid-soluble proteins contained in acid whey generated when obtaining an acid casein material which is supernatant in acid precipitation of skim milk at pH4.6 (PTL 2); a casein hydrolysate (PTL 3); and κ-casein (PTL 4) are also reported to have a GLP-1-releasing activity.

### Citation List

### Patent Literature

PTL 1: WO 01/37850
PTL 2: JP-T-2007-525404
PTL 3: JP-T-2009-517464
PTL 4: WO 2007/037413

### Non-patent Literature

NPL 1: Yabe D et al. (2011) Prog. Biophys. Mol. Biol. 107, 248-256
NPL2: Mejer JJ et al. (2002) Eur. J. Pharmacol. 440 (2-3), 269-279
NPL 3: Anna L. Gillespie et al. (2016) Food Chem. 211, 148-159
NPL 4: JC Gevrey et al. (2004) Diabetologia 47, 926-936
NPL 5: Martine Cordier-Bussat et al. (1998) Diabetes 47, 1038-1045
NPL 6: Dina Ripken et al. (2016) J. of Nutri. Biochem. 32, 142-150
NPL 7: N Higuchi et al. (2013) Endocrinology 154 (9), 3089-3098
NPL 8: Y Ishikawa et al (2015) Food Funct. 6, 2525-2534
NPL 9: Liou et al. (2011) Am J. Physiol. Gastrointest. Liver Physiol. 300 (5), 895-902
NPL 10: Diakogiannaki E et al. (2013) Diabetologia 56 (12), 2688-2696
NPL 11: Reimer et al. (2001) Endocrinology 142 (10), 4522-4528
NPL 12: Lijuan Sun et al. (2016) Br. J. Nutri 116, 1216-1221

### Summary of Invention

### Technical Problem

As components having a GLP-1-releasing activity, milk-derived components are attractive from the viewpoint of safety, ease of intake in a food or drug, impression of consumers, and the like.

However, heretofore reported milk-derived components unfortunately require complicated steps for preparation from milk, leading to high cost. In addition, the GLP-1-releasing activity of such preparations is not always sufficient and is not suitable for practical use.

In view of the above situation, an object of the present invention is to provide an excellent GLP-1 secretagogue and a composition for promoting GLP-1 secretion that contain as an active ingredient a milk-derived component that can be mass-produced in an efficient manner, preferably a composition that is suitable for food or drink or drug.

### Solution to Problem

As a result of intensive and extensive studies for achieving the above object, the present inventors have found that a micellar casein and/or a hydrolysate thereof has an excellent GLP-1-releasing activity /GLP-1 secretion stimulating activity, thereby completing the present invention.

Specifically, the present invention provides a GLP-1 secretagogue and a composition for promoting GLP-1 secretion that comprise a micellar casein and/or a hydrolysate thereof as an active ingredient. The micellar casein hydrolysate preferably has a weight average molecular weight of 400 to 5000.

Another aspect of the present invention provides a GLP-1 secretagogue and a composition for promoting GLP-1 secretion that contain a fraction of or a purified product of a micellar casein hydrolysate as an active ingredient. The fraction or the purified product of a micellar casein hydrolysate preferably contains one or two or more peptides each comprising an amino acid sequence of any one of SEQ ID NOS: 1 to 7.

Still another aspect of the present invention provides a GLP-1 secretagogue and a composition for promoting GLP-1 secretion that comprise one or two or more peptides each containing an amino acid sequence of any one of SEQ ID NOS: 1 to 7 as an active ingredient.

The composition for promoting GLP-1 secretion of the present invention is preferably in a pharmaceutical composition. In this form, the pharmaceutical composition is preferably for prevention and/or improvement of hyperglycemia, diabetes, obesity, or hyperphagia, or for suppression of postprandial blood glucose level or suppression of appetite.

Alternatively, the composition for promoting GLP-1 secretion of the present invention is preferably in a food or drink composition.

### Advantageous Effects of Invention

The present invention provides the excellent GLP-1 secretagogue and composition for promoting GLP-1 secretion . The composition for GLP-1 secretion of the present invention can be in a food or drink. Alternatively, the composition for promoting GLP-1 secretion of the present invention can be in a drug and is useful for prevention or improvement of a lifestyle-related disease, such as diabetes.

### Description of Embodiments

Next, the present invention will be described in detail. However, the present invention is not limited to the following embodiments and can be freely changed within the scope of the present invention.

### <GLP-1 Secretagogue and composition for promoting GLP-1 secretion>

The GLP-1 secretagogue and the composition for promoting GLP-1 secretion of the present invention contain a micellar casein and/or a hydrolysate thereof as an active ingredient. Note that the composition for promoting GLP-1 secretion of the present invention is also referred to as a composition for promoting GLP-1 secretion stimulation and is sometimes referred to as a GLP-1 secretagogue or a GLP-1 secretion stimulator.

In the present invention, a "micellar casein" refers to a composition containing micelle colloid formed of casein proteins, what is called casein micelle. A micellar casein is typically present in a dairy product such as a milk or a skim milk, and can be obtained, for example, by subjecting a skim milk to membrane separation. A milk protein concentrate obtained from a skim milk through the membrane separation is used as a micellar casein material. A micellar casein is obtained by membrane separation and thus is not subjected to acid precipitation and/or alkaline lysis which is performed in a production process of a caseinate, such as a casein sodium material. Accordingly, it is considered that the micellar casein maintains the "micelle structure" which is said to be originally present in raw milk.

Casein constituting a micellar casein are α-, β-, and κ-casein, and have the same composition as a casein that typically exists in milk. In addition, the micellar casein has the same form as micelle colloid of a casein that typically exists in milk and has the form of colloid particles having a mean particle size of 20 to 600 nm.

Note that the micellar casein in the present invention is distinguished from κ-casein itself which does not form micelle colloid, as shown in the Examples herein.

In addition, the micellar casein of the present invention is preferably derived from a milk and preferably derived from a cow's milk.

The micellar casein of the present invention is preferably produced from a skim milk which is obtained by removing lipids from a raw milk by a certain method. Specifically, a certain fraction of a skim milk that is obtained by removing components preferably having a particle size of 0.1 µm or less from the skim milk can be taken as the micellar casein. Typically, components having a particle size of 0.1 µm or less contained in a skim milk are whey proteins, sugar, and various other components.

Such a treatment for removing components having a particle size of 0.1 µm or less can be achieved, for example, by allowing the skim milk to pass through a microfiltration membrane, MF membrane, having a pore size of 0.1 to 0.2 µm, and components remaining on the membrane after the treatment are taken as the micellar casein. The obtained micellar casein may be subjected, as needed, to concentration, spray drying, or other treatments . In a step of preparing a micellar casein, a treatment that causes denaturation of proteins, such as an acid or alkali treatment, addition of a protease, and a heat treatment of 70°C or higher, is preferably not performed.

The thus-produced micellar casein is a casein which typically exists in milk and which maintains the composition and the form of micelle colloid. Due to the nature of operation such as the membrane treatment, the micellar casein is typically obtained in the form containing whey proteins therein, and such mixing is allowed as long as the effects of the present invention is not impaired. The amount of such proteins mixed is preferably 15% by mass or less, more preferably 10% by mass or less, and further preferably 5% by mass or less based on the whole micellar casein.

Note that the ratio of a casein to whey proteins contained in a milk is known to be typically about 8:2, and the ratio of casein to whey proteins, casein:whey proteins, in the micellar casein used in the present invention is preferably 8:2 and more preferably 9:1.

As the micellar casein of the present invention, a commercial product may be used. Preferred examples include a micellar casein manufactured using such a membrane treatment as described above by Milei Gmbh (Germany) and a micellar casein manufactured by Ingredia, for example Prodiet 87B Fluid.

It has been traditional to obtain a casein material from a skim milk, but in the traditional method, a casein material prepared from a skim milk does not maintain the form of micelle colloid and thus is distinguished from the active ingredient in the present invention.

For example, a casein has traditionally been obtained as follows: a skim milk obtained by removing lipids from a raw milk is subjected to an acid coagulation reaction with an added acid, such as sulfuric acid, or an enzyme reaction with added rennet, then is subjected to a preheat step to separate gelation proteins or a heating step to solubilize the precipitated casein by an alkali, such as sodium hydroxide, thereby preparing a casein as a caseinate. Note that a casein obtained by separating whey through an acid coagulation reaction is herein referred to as an "acid casein material". A casein obtained by subjecting an acid casein to a treatment with sodium hydroxide is herein referred to as a "casein sodium material".

The micellar casein hydrolysate which is an active ingredient of the GLP-1 secretagogue and the composition for promoting GLP-1 secretion of the present invention is preferably an protein or peptide product resulting from degradation by an enzyme, such as a protease. The enzyme used for the enzyme degradation may be any enzyme, such as a protease, and is preferably a gastric enzyme which can simulate the state of digestion that the micellar casein taken in the body undergoes in the gastrointestinal tract. Examples thereof include pepsin and pancreatin. In addition, the degradation by such a protease may be such degradation that occurs under a condition such as pH, reaction temperature, reaction time, and enzyme concentration of digestion that the micellar casein in the body typically undergoes in the gastrointestinal tract.

The enzyme degradation product of a micellar casein may be any of a crude product resulting from degradation of the micellar casein with a protease or the like, a fraction of the crude product, and a purified product thereof.

The micellar casein hydrolysate of the present invention preferably has a weight average molecular weight (Mw) of 400 to 5000, more preferably 500 to 3500, and particularly preferably 1000 to 2000. Such a hydrolysate has the size and structure of the digested product typically resulting from degradation of a micellar casein taken in the body with a protease, and is distinguished from digested products of other casein materials digested under the same condition in the size, structure, and GLP-1-releasing activity and/or GLP-1 secretion stimulating activity as described in Examples herein.

Note that the weight average molecular weights here are measured and calculated by the following method. That is, by high performance liquid chromatography, using a polyhydroxyethyl aspartamide column (from Poly LC, diameter 4.6 × 200 mm), a sample is eluted with a 20 mM sodium chloride 50 mM formic acid solution at an elution rate of 0.4 mL/min ("High Performance Liquid Chromatography of Proteins and Peptides", Special Issue of Chemistry, No. 102, edited by Nobuo Ui et al, p. 241, Kagaku Dojin (1984)). The detection is performed using a UV detector manufactured by Shimadzu Corporation and the data are analyzed with a GPC analytic system manufactured by Shimadzu Corporation to calculate a weight average molecular weight. Note that, as standard samples for calculation of molecular weights, proteins and/or peptides having known molecular weights may be appropriately used, and, for example, the reference standards used in Examples described herein can be used.

The active ingredient of the GLP-1 secretagogue and the composition for promoting GLP-1 secretion of the present invention may be a fraction or purified product of a micellar casein hydrolysate as described above. In this case, the fraction or purified product selected in the hydrolysate has a peptide fragment that is largely contained in a digested product resulting from degradation of a micellar casein taken in the body with a protease and that has a GLP-1-releasing activity and/or a GLP-1 secretion stimulating activity.

The fraction or purified product of a micellar casein hydrolysate preferably contains a peptide containing an amino acid sequence of any one of SEQ ID NOS: 1 to 7. One or two or more peptides each containing an amino acid sequence of any one of SEQ ID NOS: 1 to 7 may be contained.

The peptide containing an amino acid sequence of any one of SEQ ID NOS: 1 to 7, which has a GLP-1-releasing activity and/or GLP-1 secretion stimulating activity, may be contained in the composition for promoting GLP-1 secretion, not only in the form of a peptide contained in a micellar casein hydrolysate or in a fraction or purified product thereof, but also in the form of a peptide obtained through a chemical synthesis or biosynthesis. Accordingly, a composition for promoting GLP-1 secretion that contains one or two or more peptides each containing an amino acid sequence of any one of SEQ ID NOS: 1 to 7 as an active ingredient is also encompassed in the present invention.

Here, the peptide containing an amino acid sequence of any one of SEQ ID NOS: 1 to 7 may be contained in the form of a salt thereof. The peptide may have any one or two or more amino acids added to the C-terminal and/or N-terminal thereof as long as it contains an amino acid sequence of any of SEQ ID NOS: 1 to 7. Here, "two or more" preferably means, but is not limited to, 2 to 10.

The peptides largely exist in a micellar casein hydrolysate resulting from digestion with a protease and are peptide fragments that are presumed to contribute to promotion of GLP-1 secretion. In particular, peptides having an amino acid sequence of SEQ ID NOS: 1 to 5 are recognized to exist in a micellar casein hydrolysate but are not produced in hydrolysis of a casein. Peptides of an amino acid sequence represented by SEQ ID NOS: 6 to 7 are recognized to exist both in a micellar casein hydrolysate and in a casein hydrolysate. The difference in the presence in the hydrolysates is considered to be attributable to difference in the digestion pattern due to whether the casein micelle structure exists.

The GLP-1 secretagogue and the composition for promoting GLP-1 secretion of the present invention have a GLP-1-releasing activity and/or a secretion stimulating activity. Specifically, when the GLP-1 secretagogue or the composition for promoting GLP-1 secretion is ingested, the amount of GLP-1 secreted from intestinal endocrine cells, L cells, can be increased in the gastrointestinal tract as compared with the case where it is not ingested.

The GLP-1 secretagogue and the composition for promoting GLP-1 secretion of the present invention are thus useful for a subject of a disease or pathology that can be prevented or improving by promoting GLP-1 secretion or a disease or pathology attributable to GLP-1 hyposecretion. The GLP-1 secretagogue and the composition for promoting GLP-1 secretion can be used, for example, in a patient to prevent or improve hyperglycemia, diabetes, obesity, or hyperphagia, or can be used for appetite suppression in an alimentary therapy. In addition, the application of the GLP-1 secretagogue and the composition for promoting GLP-1 secretion of the present invention may be a therapeutic use or a non-therapeutic use. Note that the "non-therapeutic" is a concept not containing a medical practice, that is, a treatment practice through therapy for a human body.

That is, another aspect of the present invention is a method for promoting GLP-1 secretion, the method including a step of administering the GLP-1 secretagogue or the composition for promoting GLP-1 secretion of the present invention to a subject. The method may be a method of treating, improving, and/or preventing the aforementioned disease or pathology by promoting GLP-1 secretion, or may be a method of suppressing the postprandial blood glucose level increase and/or appetite of the subject.

Still another aspect of the present invention is a use of any one selected from the group consisting of a micellar casein, a hydrolysate thereof, a fraction thereof, a purified product thereof, and a peptide containing an amino acid sequence of any one of SEQ ID NOS: 1 to 7 in production of a GLP-1 secretagogue or a composition for promoting GLP-1 secretion.

Still another aspect is a use of any one selected from the group consisting of a micellar casein, a hydrolysate thereof, a fraction thereof, a purified product thereof, and a peptide containing an amino acid sequence of any one of SEQ ID NOS: 1 to 7 in promotion of GLP-1 secretion.

### <Pharmaceutical composition>

A preferred form of the composition for promoting GLP-1 secretion of the present invention is a pharmaceutical composition for promoting GLP-1 secretion.

The pharmaceutical composition may be orally or parenterally administered, but preferably is orally administered. A preferred example of parenteral administration is a direct administration to the stomach or the small intestine.

Such a pharmaceutical composition can be appropriately formulated into a desired dosage form depending on the administration method. In the case of oral administration, for example, the composition can be formulated into solid preparations, such as a powder, granules, tablets, and capsules; and liquid preparations, such as a solution, a syrup, a suspension, and an emulsion. In addition, in the case of a parenteral administration, the composition can be formulated into a suppository, an ointment, and the like.

The formulation can be appropriately implemented by a known method depending on the dosage form. In formulation, a formulation carrier may be appropriately incorporated.

The content of the micellar casein and/or hydrolysate thereof in the formulation is not limited and may be appropriately selected according to the dosage form based on the daily intake or dose. For example, in the case of an orally administered formulation, the daily intake or dose for an adult is preferably 0.1 to 4 g/kg body weight/day, more preferably 0.2 to 2 g/kg body weight/day, and further preferably 0.4 to 1.2 g/kg body weight/day in terms of the amount of proteins in the micellar casein or in terms of the amount of peptides in the micellar casein hydrolysate.

In formulation, in addition to the active ingredient of the composition for promoting GLP-1 secretion of the present invention, various pharmacologically acceptable organic or inorganic carriers can be used depending on the dosage form as long as the effects of the present invention are not impaired. Examples of carries in the case of a solid formulation include an excipient, a binder, a disintegrator, a lubricant, a stabilizer, and a corrigent. In addition, components generally used in formulation, such as a pH modifier, a colorant, and a flavor, can be incorporated.

Examples of excipients include sugar derivatives, such as lactose, sucrose, glucose, mannitol, and sorbitol; starch derivatives, such as corn starch, potato starch , α-starch, dextrin, and carboxymethyl starch; cellulose derivatives, such as crystalline cellulose, hydroxypropylcellulose, hydroxypropylmethylcellulose, carboxymethylcellulose, and carboxymethylcellulose calcium; Arabic rubber; dextran; pullulan; silicate derivatives, such as light anhydrous silicic acid, synthetic aluminum silicate, and magnesium aluminometasilicate; a phosphate derivative, such as calcium phosphate; a carbonate derivative, such as calcium carbonate; and a sulfate derivative, such as calcium sulfate.

Examples of binders include, in addition to the above excipients, gelatin; polyvinylpyrrolidone; and macrogol.

Examples of disintegrators include, in addition to the above excipients, chemically-modified starch or cellulose derivatives, such as croscarmellose sodium, carboxymethylstarch sodium, and crosslinked polyvinyl pyrrolidone.

Examples of lubricants include talc; stearic acid; metal stearates, such as calcium stearate and magnesium stearate; colloidal silica; waxes, such as veecum and spermaceti; boric acid; glycols; carboxylic acids, such as fumaric acid and adipic acid; a sodium carboxylate, such as sodium benzoate; a sulfuric acid salt, such as sodium sulfate; Leucine; lauryl sulfate salts, such as sodium lauryl sulfate and magnesium lauryl sulfate; silicates, such as silicic anhydride and silicic acid hydrate; and starch derivatives.

Examples of stabilizers include p-hyroxybenzoic acid esters, such as methylparaben and propylparaben; alcohols, such as chlorobutanol, benzyl alcohol, and phenylethyl alcohol; benzalkonium chloride; acetic anhydride; and sorbic acid.

Examples of corrigents include a sweetener, an acidulant, and a fragrance.

Note that examples of carriers used in the case of liquid agents for oral administration include water or other solvents.

The pharmaceutical composition for promoting GLP-1 secretion of the present invention has an action to promote GLP-1 secretion in the gastrointestinal tract as described above and GLP-1 has a hypoglycemic action. Thus, the timing to take the pharmaceutical composition of the present invention is not limited, but the pharmaceutical composition is preferably taken or administered, for example, at 2 hours before meals to 2 hours after meals and more preferably at 1 hour before meals to 1 hour after meals.

The pharmaceutical composition of the present invention is useful for subjects of diseases or pathologies that can be prevented or improved by promoting GLP-1 secretion or diseases or pathologies attributable to GLP-1 hyposecretion. The pharmaceutical composition may be a composition for prevention and/or improvement of, for example, hyperglycemia, diabetes, obesity, or hyperphagia. The pharmaceutical composition may also be a composition for suppression of postprandial blood glucose level increase or suppression of appetite.

Another aspect of the present invention is a use of any one selected from the group consisting of a micellar casein, a hydrolysate thereof, a fraction thereof, a purified product thereof, and a peptide containing an amino acid sequence of any one of SEQ ID NOS: 1 to 7 in production of a pharmaceutical composition for prevention and/or improvement of hyperglycemia, diabetes, obesity, or hyperphagia, or for suppression of postprandial blood glucose level or suppression of appetite.

Still another aspect is a use of any one selected from the group consisting of a micellar casein, a hydrolysate thereof, a fraction thereof, a purified product thereof, and a peptide containing an amino acid sequence of any one of SEQ ID NOS: 1 to 7 in prevention and/or improvement of hyperglycemia, diabetes, obesity, or hyperphagia, or in suppression of postprandial blood glucose level or suppression of appetite.

Still another aspect is any one selected from the group consisting of a micellar casein, a hydrolysate thereof, a fraction thereof, a purified product thereof, and a peptide containing an amino acid sequence of any one of SEQ ID NOS: 1 to 7 that is used for prevention and/or improvement of hyperglycemia, diabetes, obesity, or hyperphagia, or for suppression of postprandial blood glucose level or suppression of appetite.

### <Food or drink composition>

Another preferred form of the composition for GLP-1 secretion of the present invention is a food or drink composition for promoting GLP-1 secretion.

In this form, the GLP-1 secretagogue or the composition for promoting GLP-1 secretion of the present invention may be incorporated in a food or drink as an active ingredient or the composition for promoting GLP-1 secretion itself may be in the form of a food or drink composition.

The food or drink composition of the present invention may be in any form of liquid, paste, solid, and powder, and examples thereof include tablet confectionery, liquid diet, and feed including one for pets, as well as wheat flour products, ready-to-eat foods, processed agricultural products, processed marine products, processed livestock products, milk and dairy products, oils and fats, basic seasonings, combined seasonings and foods, frozen foods, confectionery, drinks, and other commercial products.

In the food or drink composition of the present invention, components other than the active ingredient of the composition for promoting GLP-1 secretion of the present invention, the use of which components in foods and drinks is approved under a provision of Food Sanitation Act or other food-related laws, can be incorporated with no limitation to the extent that the effects of the present invention are not impaired. For example, carbohydrates, such as dextrin and starch; proteins, such as gelatin, soy protein, and corn protein; amino acids, such as alanine, glutamine, and isoleucine; polysaccharides, such as cellulose and Arabic rubber; and oils and fats, such as soy oil and medium chain fatty acid triglycerides, can be incorporated. Incorporation of a carbohydrate is particularly preferred due to the additional beneficial effect of the present invention that nutrition can be taken while controlling blood glucose level.

In addition, the food or drink composition of the present invention may be provided or sold as a food or drink on which a health application, such as applications for preventing or treating diseases for which promotion of GLP-1 secretion is effective, is indicated.

The "indication" actions include all actions for informing users of the application, and any expression that can evoke or bring into mind the application falls into the "indication" action of the present invention regardless of the object, content, subject, and medium of the indication.

In addition, the "indication" is preferably provided in such an expression that users can directly recognize the application. Specific examples of indications include: an action to deliver, pass, exhibit for delivery or passing, or import a commodity with respect to a food or drink or a package of such a commodity with the application written thereon; and an action to exhibit or distribute advertising, a price list, or a transaction document of a commodity with the application written thereon, or to provide information of such an advertising, a price list, or a transaction document with the application included therein by an electromagnetic method such as the Internet.

On the other hand, the content of the indication is preferably an indication approved by the government or the like (for example, an indication or the like approved under various institutions established by the government and put in a manner based on the approval). The content of the indication is preferably put on a package, a container, a catalog, a pamphlet, an advertising material in the selling site, such as POP, or other documents.

Examples of "indications" also include indications of a health food, a functional food, an enteral nutritive food, a food for special uses, a health functional food, a special health food, a nutritive functional food, a functionality-indicated food, a quasi-drug, or the like. Particularly among them, indications approved by Consumer Affairs Agency, for example, indications approved by institutions about special health food, nutritive functional food, or functionality-indicated food or institutions similar to the above are mentioned. Specific examples include an indication of a special health food, an indication of a conditional special health food, an indication that the composition may influence on the body structure or function, an indication about reduction of a disease risk, and an indication about evidence-based functionality. More specifically, typical examples include an indication of a special health food, established in Cabinet Office Ordinance on Approval, etc. of Indication of Special use provided in Health Promotion Act (Cabinet Office Ordinance No . 57 of August 31, 2009) (especially an indication of a health application) and similar indications thereto.

Note that the content of the micellar casein and/or hydrolysate thereof in production of the food or drink composition is not limited and can be appropriately selected based on the daily intake. For example, the daily intake by an adult is preferably 0.1 to 4 g/kg body weight/day, more preferably 0.2 to 2 g/kg body weight/day, and further preferably 0.4 to 1.2 g/kg body weight/day in terms of the amount of proteins in the micellar casein or in terms of the amount of peptides in the micellar casein hydrolysate.

The GLP-1 secretagogue and the composition for promoting GLP-1 secretion of the present invention have an action to stimulate and promote GLP-1 secretion in the gastrointestinal tract as described above and GLP-1 has a hypoglycemic action. With the composition for promoting GLP-1 secretion of the present invention in the form of a food or drink composition, GLP-1 secretion can be promoted during meals, resulting in suppression of the glucose level increase due to meals.

Thus, the food or drink composition of the present invention is useful for subjects of diseases or pathologies that can be prevented or improved by promoting GLP-1 secretion or diseases or pathologies attributable to GLP-1 hyposecretion. For example, the food or drink composition can be used in a patient of hyperglycemia, diabetes, obesity, or hyperphagia for prevention and/or improvement thereof, and can also be used for suppression of postprandial blood glucose level increase or appetite suppression in an alimentary therapy.

Another aspect of the present invention is a use of any one selected from the group consisting of a micellar casein, a hydrolysate thereof, a fraction thereof, a purified product thereof, and a peptide containing an amino acid sequence of any one of SEQ ID NOS: 1 to 7 in production of a food or drink composition for prevention and/or improvement of hyperglycemia, diabetes, obesity, or hyperphagia, or for suppression of postprandial blood glucose level or suppression of appetite.

### Examples

The present invention will be described more specifically with respect to examples, but the present invention is not limited to the examples.

The GLP-1 secretion promoting activity (secretion stimulating activity) of the micellar casein was investigated by the following Examples.

### <Example 1> Comparison in GLP-1 secretion promoting activity (secretion stimulating activity) between micellar casein and acid casein material or casein sodium material

### (1) Preparation of samples

A micellar casein manufactured by Milei Gmbh was used as the micellar casein. An acid casein material (Lactic Casein 720, manufactured by Fonterra) and a casein sodium material (Tatua 100, manufactured by Tatua) which were prepared from a skim milk by a traditional method were provided as comparative samples.

Each sample was dissolved in Milli Q water and a solution having a protein concentration of 7.5 mg/mL was prepared by the Bradford method. The solution was adjusted to pH 2.0, which is the same pH as in the human stomach, with a 1 mol/L hydrochloric acid, then pepsin (Pep, Sigma-Aldrich; in 1 mM HCL) was added in an amount of 1/12.5 (w/w) relative to the proteins, and the mixture was shaken in a 37°C thermostat at 140 rpm for 30 minutes. Then, the pH was adjusted to 7.0 with a 1 mol/L sodium hydrogen carbonate solution to stop the digestion reaction of pepsin. Subsequently, pancreatin (Pan, Sigma-Aldrich; in 0.1 M NaHCO₃) was added in an amount of 1/62.5 (w/w) relative to the proteins, and the mixture was shaken in a 37°C thermostat at 140 rpm for a desired time (5 minutes,10 minutes, 15 minutes, 30 minutes,45 minutes, 60 minutes, 120 minutes, or overnight). After that, with heat at 90°C for 3 minutes, the digestion reaction by pancreatin was stopped. The solution after digestion was lyophilized and stored at 4°C. The digestion reactions simulated the state of digestion that a micellar casein and various casein materials taken in the body undergo in the gastrointestinal tract.

### (2) Cell assay using mouse lower gastrointestinal tract-derived gastrointestinal endocrine cell line (GLUTag cell)

GLUTag cells (see, for example, Food Funct. , 2015, vol.6, p.2525-2534) were cultured on a culture medium containing 10% fetal bovine serum (Dulbecco's modified Eagle's medium (DMEM), GIBCO) at 37°C in a 5% CO₂ incubator for 2 to 3 days into a subconfluent layer. Then, the culture medium was removed, and the cells were suspended twice with HEPES buffer (20 mM HEPES(Sigma), 140 mM NaCl (Wako), 4.5 mM KCl (Wako), 10 mM Glucose (Wako), 1.2 mM MgCl₂ (Wako), 1.2 mM CaCl₂ (Wako), 0.1% BSA (Sigma), pH7.4).

The lyophilized sample of (1) was dissolved with HEPES buffer so as to give a concentration of 5 mg/mL, and 80 µL of the resulting solution was added to each well of a 48-well culture plate containing the GLUTag cells cultured therein and the cells were incubated at 37°C for 60 minutes. After the treatment, the culture supernatant was collected and was subjected to centrifugation at 800 g at 4°C for 5 minutes and the resulting supernatant was stored at -80°C as an ELISA measurement sample. The amount of GLP-1 in the supernatant was measured using ELISA (Millipore) according to a protocol recommended by the manufacturer. The amount of GLP-1 was represented as a relative amount to 100% of that of a group treated only with HEPES buffer (blank group). The number of samples was always three (n = 3).

Table 1 shows the measurement results of the amounts of GLP-1 secreted by treating the samples. The micellar casein showed significantly higher GLP-1 secretion stimulating activity on GLUTag cells as compared with the acid casein material and casein sodium material which are existing casein materials, in all the digestion conditions. On the other hand, no significant difference was shown in the GLP-1 secretion stimulating activity between the acid casein material and the casein sodium material. The following tendency was also seen: the difference in the GLP-1 secretion stimulating activity became pronounced as the digestion of the casein proceeded. Note that the Tukey-Kramer method was used for the significance test of the data.

Note that the κ-casein sample which is demonstrated to have a GLP-1 secretion stimulating activity in PTL 4 contains the acid casein material in this Example.

**[Table 1]**

| | | GLP-1 secretion stimulating activity on GLUTag cells (unit: %, ratio to blank group (100%)) | | | | | | | | | |
|---|---|---|---|---|---|---|---|---|---|---|---|
| | | non-degraded | pep only | pep + pan5 | pep + pan10 | pep + pan15 | pep + pan30 | pep + pan45 | pep + pan60 | pep + pan120 | pep + pan O.N. |
| Micellar casein | Average | 175.0 (a) | 267.5 (a) | 262.2 (a) | 250.9 (a) | 295.1 (a) | 339.8 (a) | 385.9 (a) | 368.9 (a) | 350.0 (a) | 338.5 (a) |
| | Standard error | 41.0 | 20.9 | 25.0 | 21.5 | 16.6 | 49.8 | 31.2 | 19.9 | 32.2 | 19.7 |
| Acid casein material | Average | 117.9 (b) | 193.8 (b) | 183.7 (b) | 183.1 (b) | 198.8 (b) | 214.9 (b) | 207.7 (b) | 180.8 (b) | 108.5 (b) | 96.1 (b) |
| | Standard error | 13.6 | 17.1 | 15.0 | 11.1 | 15.2 | 10.5 | 0.2 | 21.0 | 16.8 | 27.0 |
| Casein sodium material | Average | 105.9 | 176.7 (b) | 165.9 (b) | 173.4 (b) | 185.9 (b) | 178.8 (b) | 164.5 (b) | 142.0 (b) | 94.9 (b) | 89.0 (b) |
| | Standard error | 15.5 | 16.5 | 13.7 | 4.7 | 8.5 | 4.5 | 16.0 | 20.6 | 19.7 | 20.2 |

| | | | | | | | | | | | |
|---|---|---|---|---|---|---|---|---|---|---|---|
| * Abbreviation: pep: pepsin treatment, pan: pancreatin treatment, the numeral following "pan" represents the treatment time (min). | | | | | | | | | | | |

In the same treatment group, the same alphabet in parenthesis means there is no significant difference and the different alphabets in parenthesis mean there is a significant difference of P < 0.05.

The treatment time with pep was 30 minutes in all cases.

### <Example 2> Comparison in GLP-1 secretion promoting activity (secretion stimulating activity) between micellar casein and mixture of casein sodium material and whey proteins

A micellar casein obtained by removing components having a particle size of 0.1 to 0.2 µm or lower from a skim milk by a membrane treatment was produced in the form containing approximately 10% of whey proteins together with a casein. The ratio, casein:whey proteins, by mass in the lot of the micellar casein used in Example 1 was measured to be 90.6:9.4.

Note that whey proteins are also known to have a GLP-1 secretion stimulating activity in a non-degradated and a degradated state (Food Chemistry, 189, 2015, p.120-128).

Thus, the following test was performed in order to confirm that the GLP-1 secretion stimulating activity of the micellar casein found in Example 1 was not attributable only to the whey proteins.

### (1) Preparation of samples

The micellar casein used in Example 1, and a composition in which a casein sodium material (Tatua 100, from Tatua) and a whey protein isolate (WPI, WPI8855, from Fonterra) were mixed at a ratio by mass of 90.6:9.4 were provided as samples. Each of the samples was subjected to digestion reactions with pepsin and pancreatin by the same method and conditions as in Example 1, except that the reaction time with pancreatin was 30 minutes, 60 minutes, or 120 minutes, and the solution after digestion was lyophilized and stored at 4°C.

### (2) Cell assay using mouse lower gastrointestinal tract-derived gastrointestinal endocrine cell line (GLUTag cell)

The lyophilized sample of (1) was subjected to an assay using GLUTag cells by the same method and conditions as in Example 1 and the amount of GLP-1 secreted was represented as a relative amount to 100% of that of a blank group. The number of samples was always three (n = 3).

Table 2 shows the measurement results of the amounts of GLP-1 secreted by treating the samples.

The following tendency was found: the micellar casein had a higher GLP-1 secretion stimulating activity as compared with the mixture of the casein sodium material and the whey protein isolate. Especially, the GLP-1 secretion stimulating activity was significantly higher under digestion conditions of pepsin and pancreatin 60 minutes or of pepsin and pancreatin 120 minutes. This suggests that the higher GLP-1 secretion stimulating activity of the micellar casein is not attributed only to the whey proteins of the approximately 10% which is mixed in preparation by a membrane treatment. Note that the Tukey-Kramer method was used for the significance test of the data.

**[Table 2]**

| | | GLP-1 secretion stimulating activity on GLUTag cells (unit: %, ratio to blank group) | | | | |
|---|---|---|---|---|---|---|
| | | non-degraded | pep only | pep + pan30 | pep + pan60 | pep + pan120 |
| Micellar casein | Average | 175.0 | 267.5 | 339.9 | 368.9 (a) | 350.0 (a) |
| | Standard error | 33.5 | 17.0 | 40.6 | 16.3 | 26.3 |
| Casein sodium material + whey protein isolate | Average | 97.5 | 252.7 | 263.2 | 280.2 (b) | 234.5 (b) |
| | Standard error | 1.3 | 8.2 | 12.7 | 13.5 | 8.6 |

| | | | | | | |
|---|---|---|---|---|---|---|
| * Abbreviation: pep: pepsin treatment, pan: pancreatin treatment, the numeral following "pan" represents the treatment time (min). | | | | | | |

In the same treatment group, the different alphabets in parenthesis mean there is a significant difference of P < 0.05. The treatment time with pep was 30 minutes in all cases.

### <Example 3> Comparison in GLP-1 secretion stimulating activity between micellar casein and κ-casein or κ-casein enzyme degradation product

The following test was performed in order to compare the GLP-1 secretion stimulating activity of the micellar casein with that of κ-casein which is a protein known to have a GLP-1 secretion stimulating activity, or with that of glycomacropeptide which is a κ-casein enzyme degradation product.

### (1) Preparation of samples

The micellar casein used in Example 1 and "κ-casein from bovine milk" (from Sigma-Aldrich) which is κ-casein were provided as samples. Each of the samples was subjected to digestion reactions with pepsin and pancreatin by the same method and conditions as in Example 2, and the solution after digestion was lyophilized and stored at 4°C.

### (2) Cell assay using mouse lower gastrointestinal tract-derived gastrointestinal endocrine cell line (GLUTag cell)

The lyophilized sample of (1) was subjected to an assay using GLUTag cells by the same method and conditions as in Example 1, and the amount of GLP-1 secreted was measured as a relative amount to 100% of that of a blank group, provided that the digested product of the κ-casein material sample was diluted at a concentration of 5 mg/mL or 0.5 mg/mL in dissolution in HEPES buffer. A glycomacropeptide (CGMP: from Arla) was also provided as a sample and the standard sample was dissolved as it was in HEPES buffer at a concentration of 5 mg/mL. In the assay, GLUTag cells obtained through depolarizing stimulation with 70 mM KCl were used as a positive control. The amount of GLP-1 secreted was represented as a relative amount to 100% of that of a blank group. The number of samples was always three or four (n = 3 to 4).

Table 3 shows the measurement results of the amounts of GLP-1 secreted by treating the micellar casein and the K-casein.

As a result, the micellar casein showed a significantly higher GLP-1 secretion stimulating activity as compared with that of the κ-casein, in all the digestion conditions. The following tendency was also seen: the difference was more remarkable in the artificially digested products (pep only, pep+pan30, pep+pan60 in Table 3) than in the non-degradated product. Note that the Tukey-Kramer method was used for the significance test of the data.

**[Table 3]**

| | | GLP-1 secretion stimulating activity on GLUTag cells (unit: %, ratio to blank group) | | | |
|---|---|---|---|---|---|
| | | non-degraded | pep only | pep + pan30 | pep + pan60 |
| Micellar casein 5 mg/ml | Average | 245.2 (a) | 370.0 (a) | 591.7 (a) | 386.7 (a) |
| | Standard error | 61. 8 | 47.2 | 90.6 | 143.0 |
| κ-Casein 5 mg/ml | Average | 176.5 (b) | 261.8 (b) | 225.2 (b) | 216.2 (b) |
| | Standard error | 30.8 | 42.0 | 37.8 | 47.8 |
| κ-Casein 0.5 mg/ml | Average | 115.6 (c) | 150.5 (c) | 140.9 (c) | 149.2 (b) |
| | Standard error | 16.8 | 25.0 | 21.8 | 28.8 |

| | | | | | |
|---|---|---|---|---|---|
| * Abbreviation: pep: pepsin treatment, pan: pancreatin treatment, the numeral following "pan" represents treatment time (min). | | | | | |

In the same treatment group, the different letters in parenthesis mean there is a significant difference of P < 0.05. The treatment time with pep was 30 minutes in all cases.

Table 4 shows the measurement results of the amounts of GLP-1 secreted by treating the micellar casein and the κ-casein enzyme degradation product.

As a result, the micellar casein showed a significantly higher GLP-1 secretion stimulating activity as compared with the glycomacropeptide in all the digestion conditions. Note that the Student t-test method was used for the significance test of the data.

Note that the casein hydrolysate sample which is demonstrated to have a GLP-1 secretion promoting activity (secretion stimulating activity) in PTL 3 contains the casein enzyme degradation product (glycomacropeptide) in this Example.

**[Table 4]**

| | | GLP-1 secretion stimulating activity on GLUTag cells (unit: %, ratio to blank group) | | | |
|---|---|---|---|---|---|
| | | non-degraded | pep only | pep + pan30 | pep + pan60 |
| Micellar casein | Average | 245.2*** | 370.0*** | 591.7*** | 386.7*** |
| | Standard error | 61.8 | 47.2 | 90.6 | 143.0 |
| Glycomacr opeptide | Average | 81.8 | | | |
| | Standard error | 12.0 | | | |

| | | | | | |
|---|---|---|---|---|---|
| * Abbreviation: pep: pepsin treatment, pan: pancreatin treatment, the numeral following "pan" represents treatment time (min). *** represents there is a significant difference of p < 0.001 in comparison with glycomacropeptide group. | | | | | |

The treatment time with pep was 30 minutes in all cases.

### <Example 4> Comparison in GLP-1 secretion stimulating activity between micellar casein and acid whey material

The following test was performed in order to compare the GLP-1 secretion stimulating activity of the micellar casein with that of an acid whey material containing acid-soluble proteins generated in production of an acid casein material.

### (1) Preparation of samples

The micellar casein used in Example 1 and a whey protein isolate (WPI8855, from Fonterra) as an acid whey containing acid-soluble proteins were provided as samples. Each of the samples was subjected to digestion reactions with pepsin and pancreatin by the same method and conditions as in Example 2, and the solution after digestion was lyophilized and stored at 4°C.

### (2) Cell assay using mouse lower gastrointestinal tract-derived gastrointestinal endocrine cell line (GLUTag cell)

The lyophilized sample of (1) was subjected to an assay using GLUTag cells by the same method and conditions as in Example 1, and the amount of GLP-1 secreted was represented as a relative amount to 100% of that of a blank group. The number of samples was always three (n = 3).

Table 5 shows the measurement results on the micellar casein and the acid whey material.

As a result, the micellar casein tended to have a higher GLP-1 secretion stimulating activity as compared with the acid whey material containing acid-soluble proteins. Especially, the GLP-1 secretion stimulating activity was significantly higher under the digestion conditions of pepsin and pancreatin 60 minutes or of pepsin and pancreatin 120 minutes. Note that the Tukey-Kramer method was used for the significance test of the data.

Note that the micelle casein acid-soluble protein sample which is proteins contained in the acid whey generated in production of the acid casein material in which a GLP-1 release stimulating activity is demonstrated in PTL 2 corresponds to the acid whey sample in this Example.

**[Table 5]**

| | | GLP-1 secretion stimulating activity on GLUTag cells (unit: %, ratio to blank group) | | | |
|---|---|---|---|---|---|
| | | non-degraded | pep only | pep + pan60 | pep + pan120 |
| Micellar casein | Average | 175.0 | 267.5 | 368.9 (a) | 350.0 (a) |
| | Standard error | 41.0 | 20.9 | 19.9 | 32.2 |
| Acid whey proteins | Average | 126.6 | 200.4 | 241.5 (b) | 220.3 (b) |
| | Standard error | 20.1 | 19.3 | 18.9 | 6.5 |

| | | | | | |
|---|---|---|---|---|---|
| * Abbreviation: pep: pepsin treatment, pan: pancreatin treatment, the numeral following "pan" represents treatment time (min). | | | | | |

In the same treatment group, the different letters in parenthesis mean there is a significant difference of P < 0.05. The treatment time with pep was 30 minutes in all cases.

### <Example 5> Comparison in GLP-1 secretion stimulating activity between micellar casein and cow's milk or raw milk

The following test was performed in order to compare the GLP-1 secretion stimulating activity of the micellar casein with that of a commercially-available cow's milk or raw milk.

### (1) Preparation of samples

The micellar casein used in Example 1, 4 lots of "Yotsuba cow's milk (UHT-sterilized)" as a commercially-available cow's milk, and 4 lots of a raw milk were provided as samples. Each sample was subjected to digestion reactions with pepsin and pancreatin by the same method and conditions as in Example 2, and the solution after digestion was lyophilized and stored at 4°C.

### (2) Cell assay using mouse lower gastrointestinal tract-derived gastrointestinal endocrine cell line (GLUTag cell)

The lyophilized sample of (1) was subjected to an assay using GLUTag cells by the same method and conditions as in Example 1, and the amount of GLP-1 secreted was represented as a relative amount to 100% of that of a blank group. The number of samples was always three or four (n = 3 to 4).

Table 6 shows the measurement results on the micellar casein, the commercially-available cow's milk, and the raw milk.

As a result, the micellar casein tended to have a higher GLP-1 secretion stimulating activity as compared with the commercially-available cow's milk and the raw milk. Especially, the GLP-1 secretion stimulating activity was significantly higher in the digestion condition of pepsin and pancreatin 60 minutes. Note that the Tukey-Kramer method was used for the significance test of the data.

**[Table 6]**

| | | GLP-1 secretion stimulating activity on GLUTag cells (unit: %, ratio to blank group) | | | |
|---|---|---|---|---|---|
| | | non-degraded | pep only | pep + pan60 | pep + pan120 |
| Micellar casein | Average | 245.2 | 370.0 | 591.7 (a) | 386.7 |
| | Standard error | 61. 8 | 47.2 | 90.6 | 143.0 |
| Commercially-available cow's milk | Average | 161.6 | 288.4 | 163.9 (b) | 138.7 |
| | Standard error | 20.8 | 33.2 | 20.0 | 19.1 |
| Raw milk | Average | 233.7 | 294.8 | 243.8 (b) | 171.3 |
| | Standard error | 36.9 | 52.0 | 37.8 | 34.0 |

| | | | | | |
|---|---|---|---|---|---|
| * Abbreviation: pep: pepsin treatment, pan: pancreatin treatment, the numeral following "pan" represents treatment time (min). | | | | | |

In the same treatment group, the same letter in parenthesis means there is no significant difference and the different letters in parenthesis mean there is a significant difference of P < 0.05.

The treatment time with pep was 30 minutes in all cases.

### <Example 6> Investigation of micellar casein digested product (enzyme degradation product) having GLP-1 secretion promoting activity (secretion stimulating activity)

A test was performed in order to investigate the molecular weight distributions of a micellar casein enzyme degradation product having a GLP-1 secretion promoting activity (secretion stimulating activity) and an acid casein material enzyme degradation product and a casein sodium material enzyme degradation product digested under the same condition as for the micellar casein enzyme degradation product.

### (1) Preparation of samples

The micellar casein used in Example 1 and an acid casein material enzyme degradation product and a casein sodium material enzyme degradation product (digested with pepsin 30 minutes and pancreatin 60 minutes) were dissolved in a 50 mM formic acid 20 mM sodium chloride solution at a concentration of 1 mg/ml to prepare samples.

### (2) Measurement of molecular weight distributions

The samples prepared in (1) were each analyzed by a high performance liquid chromatography, manufactured by Shimadzu Corporation, with a column filled with a poly-hydroxyethyl-aspartamide gel. The obtained chromatogram was analyzed by a GPC software, manufactured by Shimadzu Corporation, and the proportion of the enzyme degradation products was analyzed for each of molecular weight ranges.

Note that the following compounds were used as reference standards for calculating the molecular weights: IgG (molecular weight: 160,000 Dalton, from Sigma-Aldrich), lactoperoxidase (molecular weight: 93,000 Dalton, from Sigma-Aldrich), ovalbumin (molecular weight: 43,000 Dalton, from Sigma-Aldrich), chymotrypsinogen (molecular weight: 25,000, from Pharmacia), ribonuclease (molecular weight: 13,700 Dalton, from Pharmacia), insulin (molecular weight: 5, 740 Dalton, from Wako Pure Chemical), bacitracin (molecular weight: 1,427 Dalton, from Sigma-Aldrich), oxytocin (molecular weight: 1,007 Dalton, from Bachem Americas), enkephalinamide (molecular weight: 588 Dalton, from Bachem Americas), L-methionine (molecular weight: 149 Dalton, from Kyowa Hakko Bio), and L-glutamine (molecular weight: 146 Dalton, from Kyowa Hakko Bio.).

Table 7 shows the results of the molecular weight distributions of the digested products (enzyme degradation products) of the micellar casein, the acid casein material, and the casein sodium material.

As a result, the micellar casein enzyme degradation product tended to contain a larger amount of degradation products that have molecular weights of 2000 Dalton or higher as compared with the acid casein material enzyme degradation product and the casein sodium material enzyme degradation product.

The weight average molecular weights (Mw) of the enzyme degradation products were measured. While the micellar casein enzyme degradation product had a weight average molecular weight of 1554, the acid casein material enzyme degradation product had that of 742 and the casein sodium material enzyme degradation product had that of 733. Thus, the micellar casein enzyme degradation product had a weight average molecular weight of about two times of those of the acid casein material enzyme degradation product and the casein sodium material enzyme degradation product.

As described above, the micellar casein enzyme degradation product had a higher weight average molecular weight as compared with the other protein material enzyme degradation products. Thus, the following possibility was suggested: when the protein materials are subjected to artificial digestion, a structural difference may occur in peptides that are generated as degradation products and the difference causes the difference in GLP-1 secretion stimulating activity between artificially digested products. Furthermore, regarding the reason of the difference in peptides generated as degradation products, the following possibility was suggested: the difference in the methods of preparing the protein materials from a milk material may cause a difference in the thermal histories of the protein materials, resulting in different degrees of denaturation and aggregation of proteins contained in the materials, which influences the digestibility.

**[Table 7]**

| Molecular weight range (Dalton) | Micellar casein enzyme degradation product | Acid casein material enzyme degradation product | Casein sodium material enzyme degradation product |
|---|---|---|---|
| < 200 | 23.40 | 26.36 | 26.35 |
| 201-500 | 33.59 | 36.55 | 36.42 |
| 501-1000 | 16.03 | 16.09 | 16.27 |
| 1001-1200 | 4.02 | 4.07 | 4.11 |
| 1201-2000 | 7.84 | 7.43 | 7.42 |
| 2001-3500 | 7.80 | 6.84 | 6.95 |
| 3501-5000 | 3.37 | 2.11 | 2.02 |
| 5001-10000 | 1.41 | 0.50 | 0.44 |
| 10001-20000 | 0.87 | 0.04 | 0.02 |
| 20001-50000 | 1.49 | 0.01 | 0.00 |
| 50001-100000 | 0.18 | 0.00 | 0.00 |
| 100001 < | 0.01 | 0.00 | 0.00 |
| Weight average molecular weight (Mw) | 1554 | 742 | 733 |

| | | | |
|---|---|---|---|
| * The numerical values of molecular weight range represent the contents (%). | | | |

### <Example 7> Comparison in GLP-1 secretion stimulating activity between synthetic peptides

The following test was performed in order to identify the source of the effect of the high GLP-1 secretion stimulating activity of the digested product of a micellar casein.

### (1) Synthesis of peptides

Digested products of the "micellar casein" and the "composition in which a casein sodium material and a whey protein isolate were mixed" used in Example 2 were analyzed with a nano-liquid chromatograph-mass spectrometer, manufactured by Thermo Scientific, to thereby comprehensively analyze peptide fragments contained in the digested products. The present inventors used biosynthesis to synthesize, among the detected peptide fragments, 7 peptide fragments (SEQ ID NOS: 1 to 7) that were largely contained and were considered to greatly contribute to the GLP-1 secretion stimulating activity. The synthesized peptides (lyophilized products) were stored at -80°C.

### (2) Cell assay using mouse lower gastrointestinal tract-derived gastrointestinal endocrine cell line (GLUTag cell)

The synthesized peptides of (1) were each dissolved in HEPES buffer at a concentration of 1 mM or 5 mM and an assay using GLUTag cells was carried out in the same manner as in Example 1. The amount of GLP-1 secreted was represented as a relative amount to 100% of that of a blank group. The number of samples was always three (n = 3).

Table 8 shows the measurement results of the amounts of free GLP-1 secreted in the medium after treatment of the synthesized peptides. As a result, the peptide of SEQ ID NO: 1 (5 mM), the peptide of SEQ ID NO: 2 (5 mM), and the peptide of SEQ ID NO: 3 (1 mM) showed significantly higher GLP-1 secretion stimulating activities in comparison with the blank group. Note that the Dunnett t-test method was used for the significance test of the data.

**[Table 8]**

| | | GLP-1 secretion stimulating activity on GLUTag cells(unit: %, ratio to blank group) | |
|---|---|---|---|
| SEQ ID NO: 1 GPVRGPFPIIV | 1 mM | Average | 126.0 |
| | | Standard error | 21.5 |
| | 5 mM | Average | 254.8 (*) |
| | | Standard error | 21.3 |
| SEQ ID NO: 2 HIQKEDVPSE | 1 mM | Average | 141.8 |
| | | Standard error | 16.2 |
| | 5 mM | Average | 200.5 (*) |
| | | Standard error | 12.5 |
| SEQ ID NO: 3 YPVEPFTESQ | 1 mM | Average | 168.7 (*) |
| | | Standard error | 17.1 |
| | 5 mM | Average | 143.5 |
| | | Standard error | 17.9 |
| SEQ ID NO: 4 HQGLPQEVLNE | 1 mM | Average | 158.9 |
| | | Standard error | 16.3 |
| | 5 mM | Average | 115.6 |
| | | Standard error | 28.2 |
| SEQ ID NO: 5 LEDSPEVIESPPEINT | 1 mM | Average | 133.9 |
| | | Standard error | 6.7 |
| | 5 mM | Average | 136.9 |
| | | Standard error | 22.1 |
| SEQ ID NO: 6 SRYPSYGLN | 1 mM | Average | 123.6 |
| | | Standard error | 24.8 |
| | 5 mM | Average | 149.7 |
| | | Standard error | 18.3 |
| SEQ ID NO: 7 YQKFPQYL | 1 mM | Average | 160.8 |
| | | Standard error | 15.6 |
| | 5 mM | Average | 87.1 |
| | | Standard error | 9.4 |

| | | | |
|---|---|---|---|
| (*) represents there was a significant difference of P < 0.05 in comparison with blank group. | | | |

## Claims

1. A composition for promoting GLP-1 secretion comprising a micellar casein and/or a hydrolysate thereof as an active ingredient.

2. The composition for promoting GLP-1 secretion according to claim 1, wherein the micellar casein hydrolysate has a weight average molecular weight of 400 to 5000.

3. A composition for promoting GLP-1 secretion comprising a fraction or a purified product of a micellar casein hydrolysate as an active ingredient.

4. The composition for promoting GLP-1 secretion according to claim 3, wherein the fraction or the purified product of a micellar casein hydrolysate comprises one or two or more peptide(s) each comprising an amino acid sequence of any one of SEQ ID NOs: 1 to 7.

5. A composition for promoting GLP-1 secretion comprising one or two or more peptide(s) each comprising an amino acid sequence of any one of SEQ ID NOS: 1 to 7 as active ingredients.

6. The composition for promoting GLP-1 secretion according to any one of claims 1 to 5, wherein the composition is a pharmaceutical composition.

7. The composition for promoting GLP-1 secretion according to any one of claims 1 to 5, wherein the composition is a food or drink composition.

8. The composition for promoting GLP-1 secretion according to claim 6 or 7, wherein the composition is for prevention and/or improvement of hyperglycemia, diabetes, obesity, or hyperphagia, or for suppression of postprandial blood glucose level or suppression of appetite.

9. A use of any one selected from the group consisting of a micellar casein, a hydrolysate thereof, a fraction thereof, a purified product thereof, and a peptide comprising an amino acid sequence of any one of SEQ ID NOs : 1 to 7 in production of a food or drink composition for prevention and/or improvement of hyperglycemia, diabetes, obesity, or hyperphagia, for suppression of postprandial blood glucose level or for suppression of appetite.

10. A use of any one selected from the group consisting of a micellar casein, a hydrolysate thereof, a fraction thereof, a purified product thereof, and a peptide comprising an amino acid sequence of any one of SEQ ID NOs : 1 to 7 in production of a pharmaceutical composition for prevention and/or improvement of hyperglycemia, diabetes, obesity, or hyperphagia, for suppression of postprandial blood glucose level or for suppression of appetite.

11. A composition comprising any one selected from the group consisting of a micellar casein, a hydrolysate thereof, a fraction thereof, a purified product thereof, and a peptide comprising an amino acid sequence of any one of SEQ ID NOs: 1 to 7 used for promotion of GLP-1 secretion, for prevention and/or improvement of hyperglycemia, diabetes, obesity, or hyperphagia, for suppression of postprandial blood glucose level, or for suppression of appetite.

12. A method of preventing and/or improving a disease or a pathological condition that can be prevented or improved by promoting GLP-1 secretion or a disease or a pathological condition attributable to GLP-1 hyposecretion, wherein the method comprises a step of administering any one selected from the group consisting of a micellar casein, a hydrolysate thereof, a fraction thereof, a purified product thereof, and a peptide comprising an amino acid sequence of any one of SEQ ID NOs: 1 to 7 to a subject.

13. The method according to claim 12, wherein the disease or the pathological condition is hyperglycemia, diabetes, obesity, or hyperphagia.


SEQUENCE LISTING

<110>  Morinaga milk industry co., ltd.

<120>  GLP-1 secretagogue and composition

<130>  OP-18030-PCT

<160>  7

<170>  PatentIn version 3.5

<210>  1
<211>  11
<212>  PRT
<213>  Artificial Sequence

<220>
<223>  peptide 1

<400>  1

Gly Pro Val Arg Gly Pro Phe Pro Ile Ile Val
1               5                   10


<210>  2
<211>  10
<212>  PRT
<213>  Artificial Sequence

<220>
<223>  peptide 2

<400>  2

His Ile Gln Lys Glu Asp Val Pro Ser Glu
1               5                   10


<210>  3
<211>  10
<212>  PRT
<213>  Artificial Sequence

<220>
<223>  peptide 3

<400>  3

Tyr Pro Val Glu Pro Phe Thr Glu Ser Gln
1               5                   10


<210>  4
<211>  11
<212>  PRT
<213>  Artificial Sequence

<220>
<223>  peptide 4

<400>  4

His Gln Gly Leu Pro Gln Glu Val Leu Asn Glu
1               5                   10

<210>  5
<211>  16
<212>  PRT
<213>  Artificial Sequence

<220>
<223>  peptide 5

<400>  5

Leu Glu Asp Ser Pro Glu Val Ile Glu Ser Pro Pro Glu Ile Asn Thr
1               5                   10                  15

<210>  6
<211>  9
<212>  PRT
<213>  Artificial Sequence

<220>
<223>  peptide 6

<400>  6

Ser Arg Tyr Pro Ser Tyr Gly Leu Asn
1               5

<210>  7
<211>  8
<212>  PRT
<213>  Artificial Sequence

<220>
<223>  peptide 7

<400>  7

Tyr Gln Lys Phe Pro Gln Tyr Leu
1               5